(19) Europäisches Patentamt
European Patent Office
Office européen des brevets

(11) **EP 3 859 573 A1**

(12) **EUROPÄISCHE PATENTANMELDUNG**

(43) Veröffentlichungstag:
**04.08.2021 Patentblatt 2021/31**

(51) Int Cl.:
*G06F 21/35* (2013.01)     *H04L 9/32* (2006.01)
*H04L 29/06* (2006.01)     *H04W 12/08* (2021.01)
*G01N 35/00* (2006.01)     *G06F 21/62* (2013.01)
*A61B 5/15* (2006.01)

(21) Anmeldenummer: **20154624.9**

(22) Anmeldetag: **30.01.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare Diagnostics Products GmbH**
**35041 Marburg (DE)**

(72) Erfinder:
• **Eichert, Christof**
  **61462 Koenigstein (DE)**
• **Golriz, Reza**
  **55129 Mainz (DE)**
• **Preidel, Markus**
  **60320 Frankfurt (DE)**

(54) **VERFAHREN ZUM AUTOMATISCHEN ENTSPERREN ODER SPERREN EINES COMPUTERGESTÜTZTEN MEDIZINPRODUKTS**

(57) Verfahren zum automatischen Entsperren und/oder Sperren eines computergestützten Medizinprodukts in einem System, das System umfassend das computergestützte Medizinprodukt und ein mobiles Endgerät, wobei das mobile Endgerät eine erste drahtlose Kommunikationsvorrichtung zum drahtlosen Senden und Empfangen von Daten umfasst und wobei mittels des mobilen Endgeräts eine mobile Anwendungssoftware ausführbar ist, wobei das Medizinprodukt einen Gerätecomputer umfasst und wobei der Gerätecomputer eine zweite drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung des Medizinprodukts eine drahtlose Kommunikationsverbindung zu der ersten Kommunikationsvorrichtung des mobilen Endgeräts aufbauen kann, wobei das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der zweiten Kommunikationsvorrichtung das Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der Kommunikationsvorrichtung das Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

FIG 4

**Beschreibung**

Die Erfindung betrifft ein Verfahren zum automatischen Entsperren und/oder Sperren eines computergestützten Medizinprodukts in einem System, das System umfassend das computergestützte Medizinprodukt und ein mobiles Endgerät.

Heutige computergestützte Medizinprodukte, wie z.B. Analysegeräte, wie sie routinemäßig in der Analytik, der Forensik, der Mikrobiologie und der klinischen Diagnostik Verwendung finden, sind in der Lage, eine Vielzahl von Nachweisreaktionen und Analysen mit einer Vielzahl von Proben durchzuführen. Um eine Vielzahl von Untersuchungen automatisiert durchführen zu können, sind diverse automatisch arbeitende Vorrichtungen zum räumlichen Transfer von Messzellen, Reaktionsgefäßen und Reagenzflüssigkeitsbehältern erforderlich, wie z.B. Transferarme mit Greiffunktion, Transportbänder oder drehbare Transporträder, sowie Vorrichtungen zum Transfer von Flüssigkeiten, wie z.B. Pipettiervorrichtungen. Die Geräte umfassen eine zentrale Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Besonders verbreitet sind Messsysteme, die auf photometrischen (z.B. turbidimetrischen, nephelometrischen, fluorometrischen oder luminometrischen) oder radiometrischen Messprinzipien beruhen. Diese Verfahren ermöglichen den qualitativen und quantitativen Nachweis von Analyten in flüssigen Proben, ohne zusätzliche Trennschritte vorsehen zu müssen. Die Bestimmung klinisch relevanter Parameter, wie zum Beispiel der Konzentration oder der Aktivität eines Analyten erfolgt vielfach, indem ein Aliquot, also eine Teilmenge, einer Körperflüssigkeitsprobe eines Patienten mittels einer Pipettiervorrichtung aus einem Probengefäße entnommen und in ein Reaktionsgefäß transferiert wird. Das Aliquot der Probe wird dann ebenfalls mittels einer Pipettiervorrichtung mit einem oder mehreren Testreagenzien in dem Reaktionsgefäß vermischt. Die erforderlichen Reagenzien werden in einer Anzahl von Reagenzbehältern vorrätig gehalten, die wiederum in einem Reagenzbehältervorrat aufbewahrt werden. Durch das Vermischen der Probe mit dem oder den erforderlichen Reagenzien wird eine biochemische Reaktion in Gang gesetzt, die eine messbare Veränderung einer optischen Eigenschaft des Reaktionsansatzes bewirkt.

Das Messergebnis wird von dem Messsystem wiederum in eine Speichereinheit weitergeleitet und ausgewertet. Anschließend liefert das Analysegerät einem Benutzer über ein Ausgabemedium, wie z.B. einen Monitor, einen Drucker oder eine Netzwerkverbindung probenspezifische Messwerte.

Computergestützte Medizinprodukte generieren und/oder verarbeiten häufig sehr sensible persönliche Gesundheitsdaten, wie z.B. Daten zur Krankengeschichte, Laborwerte aus Körperflüssigkeiten oder Daten zur Erbinformation, sowie weitere personenbezogene Daten, wie z.B. Namen, Geburtstag, Geschlecht oder Alter. Häufig sind die Daten dabei auch untereinander verknüpft, so dass sich ganz besonders sensible Datensätze ergeben, für die besondere technische Schutzmaßnahmen nötig sind, um z.B. unbefugten Zugriff oder Manipulationen möglichst sicher auszuschließen.

In der Medizintechnik gewinnt Cyber-Security und Datenschutz insgesamt eine immer wichtigere Rolle. Dies spiegelt sich auch in teilweise zunehmend strengeren gesetzliche Auflagen wider, beispielhaft wie etwa in der Verordnung (EU) 2016/679 des Europäischen Parlaments und des Rates vom 27. April 2016 zum Schutz natürlicher Personen bei der Verarbeitung personenbezogener Daten, zum freien Datenverkehr und zur Aufhebung der Richtlinie 95/46/EG (Datenschutz-Grundverordnung), u.a. um den Datenschutz im Umgang mit Patientendaten zu stärken.

Im oft arbeitsverdichteten Alltag in Laboren, Krankenhäusern und Praxen, etc. wird jedoch teilweise beobachtet, dass Mitarbeiterinnen und Mitarbeiter beim Betrieb von Medizinprodukten z.B. zur Vereinfachung von Arbeitsschritten und zur Zeitersparnis sogar auf grundlegende Sicherheitsmaßnahmen, wie z.B. das Sperren von Computern von Medizinprodukten durch ein Passwort bei kurzfristiger Abwesenheit verzichten und sich über entsprechende Arbeitsanweisungen hinwegsetzen. Hierdurch können unbefugte Personen leicht Zugriff auf sensible Daten bekommen und es kann darüber hinaus Manipulationsgefahr der Daten bestehen, was schlimmste medizinische Folgen für die betroffenen Patienten haben kann. Weiter können sich für die handelnden Personen und die Einrichtungen erhebliche rechtliche und finanzielle Risiken ergeben, wenn verbindliche Standards nicht immer eingehalten werden.

Derzeit erfolgt die Sperrung und/oder Entsperrung von computergestützten Systemen im Bereich der Medizinprodukte manuell über die Eingabe von entsprechenden Passwörtern.

Es ist daher Aufgabe der Erfindung, manipulationssichere Verfahren und Mittel bereit zu stellen, welche es im Alltag ermöglichen, erhöhte Anforderungen an den Datenschutz beim Betreib von computergestützten Medizinprodukten zuverlässig umzusetzen und die Datenintegrität sicherzustellen.

Diese Aufgabe wird erfindungsgemäß dadurch gelöst, dass Technologien mit einer mobilen Anwendung für ein Verfahren kombiniert werden und das Sperren und/oder Entsperren eines computergestützten Medizinproduktes soweit automatisiert wird, dass diese notwendige Sicherheitsmaßnahme zum Datenschutz von Anwendern als selbstverständlich angesehen wird.

Dies hat den Vorteil, dass sehr zuverlässig erhöhte Anforderungen an den Datenschutz beim Betreib von computergestützten Medizinprodukten umsetzbar sind und die Datenintegrität sichergestellt ist. Gleichzeitig wird der Arbeitsablauf beim Betrieb der Medizinprodukte erheblich vereinfacht und beschleunigt und die Wahrscheinlichkeit eines möglichen unautorisierten Zugriffs auf Patientendaten verringert. Weiter ist es erfindungsgemäß auch möglich, sehr schnell auf eine beliebige Anzahl von mehreren Medizingeräten gleichzeitig und/oder nacheinander zugreifen zu können, was ebenfalls den Arbeitsablauf vereinfacht und beschleunigt und somit die Effizienz erhöht. Dabei wird erfindungsgemäß zur Ausführung der mobilen Anwendung bevorzugt kein spezielles mobiles Endgerät benötigt, sondern es kann z.B. ein handelsübliches Smartphone, etc. verwendet werden, was die Implementierung vereinfacht und die Akzeptanz bei den Akteuren erhöht. Es wird also, insbesondere bei stationären Geräten, eine schnelle, nutzerfreundlichen Entsperrung und/oder Sperrung ermöglicht, wodurch insbesondere die Nutzbarkeit der Geräte erhöht wird und eine Verbesserung der Sicherheit erreicht wird, auch da die Hürde zur tatsächlichen Nutzung von Sperrfunktionen im Arbeitsalltag deutlich abgesenkt wird.

Gegenstand der vorliegenden Erfindung ist also insbesondre ein Verfahren zum automatischen Entsperren und/oder Sperren eines computergestützten Medizinprodukts in einem System, das System umfassend das computergestützte Medizinprodukt und ein mobiles Endgerät, wobei das mobile Endgerät eine erste drahtlose Kommunikationsvorrichtung zum drahtlosen Senden und Empfangen von Daten umfasst und wobei mittels des mobilen Endgeräts eine mobile Anwendungssoftware ausführbar ist, wobei das Medizinprodukt einen Gerätecomputer umfasst und wobei der Gerätecomputer eine zweite drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung des Medizinprodukts eine drahtlose Kommunikationsverbindung zu der ersten drahtlosen Kommunikationsvorrichtung des mobilen Endgeräts aufbauen kann, wobei das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der zweiten Kommunikationsvorrichtung das Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der Kommunikationsvorrichtung das Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

Die Erfindung basiert dabei auf der Überlegung, dass eine speziell entwickelte mobile Anwendung auf dem mobilen Endgerät des Benutzers ausgeführt wird, wobei es sich bei dem mobilen Endgerät vorteilhafterweise um ein Smartphone, ein Tablet und/oder eine Smartwatch handeln kann. Der Benutzer meldet sich bevorzugt vorab mit einem Benutzernamen und einem Passwort bei einem dafür vorgesehenen Authentifizierungsserver an und ist über die mobile Anwendung mit dem Zielsystem, z.B. dem Geräterechner, gekoppelt. Der Geräterechner verfügt dabei über eine Senderhardware, bevorzugt für eine der bekannten drahtlosen Technologien, wie z.B. NFC (Near Field Communication), BLE (Bluetooth Low Energy) und/oder Wi-Fi. Auf dem Geräterechner wird eine dedizierte Treibersoftware im Hintergrund ausgeführt, die mithilfe der verfügbaren drahtlosen Technologie das Gerät für die mobile Anwendung sichtbar macht. Hierbei wird bevorzugt ein spezielles Geräteidentifikationstoken bereitgestellt, welches die mobile Anwendung zum Entsperren des Benutzers benötigt. Die Signalstärke wird ausgewertet, um festzustellen, ob sich ein Benutzer in der Nähe befindet. Dadurch wird sichergestellt, dass der Geräterechner nur entsperrt wird, wenn sich der Benutzer z.B. in einem vordefinierten Abstand befindet. Eine Sperrung erfolgt gegebenenfalls vorteilhafterweise ebenfalls nach einem entsprechendem Muster. Bevorzugt ist weiter ein Authentifizierungsserver vorgesehen, der für die Verwaltung der Benutzer und Geräte verantwortlich ist. Der Benutzer sowie die dedizierte Treibersoftware melden sich bei dieser Instanz an. Weiter kann dann die Entsperrung des Geräterechners über diesen Server erfolgen.

In einer bevorzugten Ausführungsform des erfindungsgemäßen Verfahrens umfasst das System weiter einen Authentifizierungsdienst, wobei eine drahtlose Kommunikationsverbindung das mobilen Endgeräts mit der ersten drahtlosen Kommunikationsvorrichtung zu dem Authentifizierungsdienst aufbaubar ist und wobei eine Kommunikationsverbindung zwischen dem Gerätecomputer des Medizinprodukts und dem Authentifizierungsdienst aufbaubar ist und wobei die Entsperrung und/oder Sperrung des Medizinprodukts mittels des Authentifizierungsdienst erfolgt, wobei der Authentifizierungsdienst bevorzugt einen Authentifizierungsserver umfasst oder ein Authentifizierungsserver ist. Dies hat den Vorteil, dass entsprechende Authentifizierungsmerkmale wie etwa Benutzernamen und Passwörter zentral über den Authentifizierungsdienst verwaltete werden können. Gelichzeitig ist eine besonders einfache und effiziente Umsetzung eines erfindungsgemäßen Verfahrens möglich. Bevorzugt erfolgt dabei eine Authentifizierung des mobilen Endgeräts und/oder des Gerätecomputers des Medizinprodukts bei dem Authentifizierungsdienst.

Bevorzugt erfolgt die Authentifizierung des mobilen Endgeräts über eine drahtlose Kommunikationsverbindung das mobilen Endgeräts mit der ersten drahtlosen Kommunikationsvorrichtung zu dem Authentifizierungsdienst. Dies ermöglicht die besonders einfache Realisierung eines erfindungsgemäßen Verfahrens.

Bevorzugt erfolgt die Authentifizierung des Gerätecomputers des Medizinprodukts über eine Kommunikationsverbindung des Gerätecomputers des Medizinprodukt zu dem Authentifizierungsdienst.

Bevorzugt umfasst das mobile Endgerät ein Smartphone oder einen tragbaren Computer, bevorzugt einen Tablett Computer, oder das mobile Endgerät ist ein Smartphone oder ein tragbarer Computer, bevorzugt ein Tablett Computer. Bevorzugt kann das mobile Endgerät auch eine Smartwatch sein oder eine Smartwatch umfassen. Dies hat den Vorteil, dass als mobiles Endgerät weit verbreitete Geräte verwendet werden können, die häufig bereits aus anderen Gründen, wie z.B. der telefonischen Erreichbarkeit oder der Zugangsmöglichkeit ins Internet von den Bedienern der Medizinprodukte bei sich getragen werden. Dies macht es besonders einfach und kostengünstig, erfindungsgemäße Verfahren umzusetzen. Auch erhöht dies die Akzeptanz bei den Bedienern, da keine weiteren Geräte benötigt werden.

Vorteilhafterweise kann insbesondere auch eine Kombination mehrerer mobiler Geräte wie Mobiltelefone und Smartwatches verwendet werden. Dabei ist vorteilhaft, dass solche Kombinationen jederzeit konfiguriert und direkt an Kundenbedürfnisse angepasst werden können.

Bevorzugt erfolgt das automatische Entsperren und/oder Sperren des computergestützten Medizinprodukts mittels eines vorbestimmten mobilen Endgeräts jeweils nur innerhalb eines vorbestimmten Zeitraums. Bevorzugt handelt es sich bei dem vorbestimmten Zeitraum z.B. um eine Arbeitsschicht oder z.B. 8, 12, oder 24 Stunden. Dies hat den Vorteil, dass sich der Benutzer zu Beginn seiner Arbeitsschicht einmal entsprechend mit seinen Zugangsdaten anmelden kann und dann während der gesamten Schicht die automatische Entsperr- bzw. Sperrfunktion nutzen kann. Nach Ende der Arbeitsschicht wird die automatische Entsperr- bzw. Sperrfunktion wieder automatisch gesperrt. Dies erhöht die Sicherheit des Systems und beugt Missbrauch z.B. mittels verlorengegangener oder verlegter mobiler Endgeräte durch nicht oder nicht mehr autorisierte Personen vor.

Bevorzugt ist das das Medizinprodukt ortsfest bzw. stationär angeordnet und nicht unmittelbar portabel wie etwa ein mobiles Smartphone, dass ein Nutzer stets unmittelbar bei sich am Körper oder in der Kleidung tragen kann.

Bevorzugt umfasst das Medizinprodukt einen automatischen Analysator, bevorzugt einen in-vitro Analysator für medizinische Proben, bevorzugt für Blut. Besonders bevorzugt handelt es sich bei dem Medizinprodukt um einen automatischen Analysator, bevorzugt einen in-vitro Analysator für medizinische Proben, bevorzugt für Blut. Dies ermöglicht es, erhöhte Datenschutzerfordernisse sehr zuverlässig auch in Bereichen wie z.B. medizinischen Laboren sicher umsetzen zu können.

Bevorzugt umfasst das Medizinprodukt ein Point-of-Care System. Bei Point-of-Care Systemen handelt es sich z.B. um Systeme zur medizinischen Testung am Ort der Versorgung oder in Nähe des Ortes der Versorgung eines Patienten. Die Testung erfolgt also am Ort der Patientenversorgung und bevorzugt gleichzeitig zur Patientenversorgung, beispielsweise in einem Behandlungszimmer in einer Arztpraxis, einem Krankenhaus, einem Geburtshaus oder z.B. etwa bei der Notfallversorgung am Ort eines Unfalls oder in einem Rettungswagen oder Rettungshubschrauber während des Krankentransports. Alternativ kann die Versorgung des Patienten mittels eines Point-of-Care Systems auch in Wohnräumen des Patienten oder an jedem andern Ort erfolgen, an dem sich der Patient zum Zeitpunkt der Versorgung aufhält. Point-of-Care Systeme können also insbesondere auch außerhalb von speziellen medizinischen Laboren oder Zentrallaboren in unmittelbarer räumlicher Nähe zum Patienten eingesetzt werden und Testungen können in Echtzeit während oder in zeitlicher Nähe zu sonstigen Untersuchungen oder Therapien erfolgen. Dabei ist das Point-of-Care System bevorzugt ortsfest bzw. stationär angeordnet und nicht unmittelbar portabel wie etwa ein mobiles Smartphone, dass ein Nutzer stets unmittelbar bei sich am Körper oder in der Kleidung tragen kann.

Bevorzugt umfasst das Medizinprodukt ein System zur Intensivpflege. Systeme zur Intensivpflege kommen z. B. im Rahmen der Intensivmedizin zum Einsatz, z.B. auf Intensivstation von Krankenhäusern oder speziell ausgestatteten Fahrzeugen oder Flugzeugen zum Krankentransport. Bevorzugt handelt es sich bei dem System zur Intensivpflege um eine Spritzenpumpe und/oder Infusionspumpe.

Bevorzugt umfasst das Medizinprodukt ein System zur medizinischen Bildgebung. Bevorzugt erfolgt die Bildgebung bei einem System zur medizinischen Bildgebung z. B. mittels Röntgenstrahlung (z. B. Röntgenaufnahmen, Durchleuchtung, Computertomographie), Radionukliden (z. B. Szintigraphie, Positronen-Emissions-Tomographie, Single-Photon-Emissionscomputertomographie), Ultraschall (z. B. Sonographie, Farbdoppler), Kernspinresonanz (z. B. Magnetresonanztomographie), Infrarotstrahlung (z. B. diagnostische Thermographie), Impedanz (z. B. Elektrische Impedanz-Tomographie) und/oder sichtbarem Licht (z. B. Endoskopie, optische Tomographie, Videorasterstereographie). Bevorzugt entspricht der erste Grenzwert einem ersten Abstand zwischen dem mobile Endgerät und dem Medizinprodukt oder ist einem solchen zugeordnet, wobei der erste Abstand bevorzugt weniger als 10 Meter, besonders bevorzugt weniger als 5 Meter beträgt. Bevorzugt hängt der erste Grenzwert und/oder der erste Abstand dabei von der Größe des Medizingeräts ab. Bevorzugt ist der erste Grenzwert umso kleiner, je größer das Medizingerät ist. Entsprechend hängt bevorzugt der erste Abstand von der Größe des Medizingeräts ab, wobei bevorzugt der Abstand umso größer ist, je größer des Medizingerät ist. Z.B. ist der erste Abstand bei kleineren Point of Care Geräten, die z.B. in der Hand gehalten werden können oder am Bett des Patienten stehen bevorzugt vergleichsweise klein und beträgt z.B. nur wenige Meter oder sogar weniger als ein Meter. Z.B. ist der erste Abstand bei großen Automatisierungsstraßensystemen und größeren Hochdurchsatzanalysatoren in einem Zentrallabor bevorzugt vergleichsweise groß und beträgt z.B. 10 Meter oder mehr. Dies ermöglicht es, die jeweiligen Grenzwerte bzw. Abstände so zu wählen, dass sie an die Erfordernisse der jeweiligen Medizingeräte und deren Bedienung angepasst sind.

Bevorzugt entspricht der zweite Grenzwert einem zweiten Abstand zwischen dem mobile Endgerät und dem Medizinprodukt oder ist einem solchen zugeordnet, wobei der zweite Abstand bevorzugt weniger als 10 Meter, besonders bevorzugt weniger als 5 Meter beträgt.

Bevorzugt wird der durch die zweite Kommunikationsvorrichtung das Medizinprodukts empfangenen Signalstärke des Signals der ersten Kommunikationsvorrichtung des mobilen Endgeräts jeweils ein entsprechender Abstand zwischen dem mobile Endgerät und dem Medizinprodukt zugeordnet.

Bevorzugt erfolgt die Zuordnung zwischen dem ersten bzw. dem zweiten Grenzwert und dem ersten bzw. zweiten Abstand zwischen dem mobile Endgerät und dem Medizinprodukt nach folgender Abschätzung für den Abstand:

$$\text{Abstand} = 10\ \text{EXP}((\text{Signalstärke} - \text{RSSI})/(10\ \text{N})),$$

wobei EXP(X) für 10 hoch X steht, Signalstärke für die jeweilig gemessene Signalstärke, RSSI für Received Signal Strength Indicator und N für eine Konstante zur effektiven Berücksichtigung von Umwelteinflüssen, wobei N typischerweise Werte von 2 bis 4, bevorzugt den Wert 2 annimmt.

Bevorzugt beträgt der erste Grenzwert -60 dB bis -70 dB, besonders bevorzugt -60 dB. Der letztere Wert entspricht dabei z.B. einem ersten Abstand von etwa einem Meter gemäß der obigen Abschätzung für den Abstand.

Dabei hängt die gemessene Signalstärke neben dem Abstand auch von der Sendestärke (Broadcasting Power value) ab. Bei maximaler Sendestärke (+4 dBm) beträgt der Wert für RSSI ca. -26 (bei einem Abstand von wenigen Centimetern) bis zu ca. - 100 (bei einem Abstand von ca. 40 bis 50 Metern).

Bevorzugt ist das erste Zeitintervall bevorzugt kleiner als 3 Minuten, besonders bevorzugt kleiner als 1 Minute. Dies hat den Vorteil, dass bei Überschreiten der entsprechenden Zeitdauer eine automatische Sperrung erfolgt. Gleichzeitig ermöglicht es jedoch, dass bei sehr kurzen Unterbrechungen der Verbindung aufgrund von z.B. kurzfristig auftretenden Störsignalen keine Sperrung erfolgt und der Betrieb somit robuster und weniger Störanfällig ist.

Bevorzugt ist der erste Grenzwert kleiner als der zweite Grenzwert. Dies hat den Vorteil, dass das System weniger Störanfällig ist und unbeabsichtigte, rasch aufeinanderfolgende Sperr- bzw. Endsperrvorgänge vermieden werden.

Bevorzugt umfasst das Sperren die Blockierung einer graphischen Bedienoberfläche einer elektronischen Anzeigevorrichtung des Medizinproduktes und/oder die Blockierung einer Benutzerschnittstelle des Medizinproduktes für die Eingabe von Befehlen. Dies hat den Vorteil, dass die angezeigten Daten, insbesondre sensible Patientendaten, für unbefugte Dritte nicht unbeaufsichtigt einsehbar sind bzw. manipuliert werden können.

Bevorzugt umfasst das Entsperren die Freigabe einer graphischen Bedienoberfläche einer elektronischen Anzeigevorrichtung des Medizinproduktes und/oder die Freigabe einer Benutzerschnittstelle des Medizinproduktes für die Eingabe von Befehlen. Dies hat den Vorteil, dass die Anzeigevorrichtung bzw. Benutzerschnittstelle ohne weitere Maßnahmen, wie z.B. die Eingabe eines Passworts, dem Nutzer unmittelbar zur Verfügung steht und z.B. Wartezeiten vermieden werden und somit der Arbeitsablauf insgesamt beschleunigt wird.

Eine weitere vorteilhafte erfindungsgemäße Lösung umfasst, dass durch Pairing ein mobiles Endgerät oder mehrere mobile Endgeräte einmalig angelernt und fest konfiguriert werden, teilweise ähnlich der aus der Automobilindustrie bekannten, sogenannten "Keyless GoTechnologie". Vorteilhafterweise kann es sich dabei erfindungsgemäß bei dem mobilen Endgerät auch um spezielle Transponder, wie z.B. in entsprechenden Autoschlüsseln verbaut, handeln. Jedoch sind erfindungsgemäß vorteilhafterweise solche speziellen Voraussetzungen an zusätzliche Hardware nicht erforderlich.

Ein weiterer Gegenstand der Erfindung ist ein Medizinprodukt umfassend einen Gerätecomputer, wobei der Gerätecomputer eine erste drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung das Medizinprodukts eine drahtlose Kommunikationsverbindung zu einer ersten drahtlose Kommunikationsvorrichtung eines mobilen Endgeräts aufbauen kann, wobei das mobile Endgerät die erste drahtlose Kommunikationsvorrichtung zum drahtlosen Senden und Empfangen von Daten umfasst und wobei mittels des mobilen Endgeräts eine mobile Anwendungssoftware ausführbar ist, wobei der Gerätecomputer so konfiguriert ist, dass das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der zweiten Kommunikationsvorrichtung das Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der Kommunikationsvorrichtung das Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

Bevorzugt umfasst das Medizinprodukt einen automatischen Analysator, bevorzugt einen in-vitro Analysator für medizinische Proben, bevorzugt für Blut. Besonders bevorzugt handelt es sich bei dem Medizinprodukt um einen automatischen Analysator, bevorzugt einen in-vitro Analysator für medizinische Proben, bevorzugt für Blut.

Bevorzugt umfasst das Medizinprodukt ein Point-of-Care System und/oder ein System zur Intensivpflege und/oder ein System zur medizinischen Bildgebung. Bevorzugt handelt es sich bei dem System zur Intensivpflege um eine Spritzenpumpe und/oder Infusionspumpe.

Ein weiterer Gegenstand der Erfindung ist die Verwendung eines erfindungsgemäßen Verfahrens in einem System, das System umfassend ein computergestützte Medizinprodukt und ein mobiles Endgerät.

Bei dem Medizingerät kann es sich bevorzugt insbesondre um ein Diagnosesystem für bildgebende Verfahren oder in-vitro Diagnosegeräte handeln.

Ausführungsbeispiele der Erfindung werden anhand von Zeichnungen näher erläutert. Darin zeigen:

FIG 1    eine schematische Darstellung eines computergestützten Medizinprodukts,

FIG 2    eine schematische Darstellung eines Systems umfassend ein computergestütztes Medizinprodukt, eine Authentifizierungsdienst und ein mobiles Endgerät,

FIG 3    ein Ablaufdiagramm eines Verfahrens zur Gerätekopplung, und

FIG 4    ein Ablaufdiagramm eines Verfahrens zum Entsperren eines Geräterechners eines computergestützten Medizinproduktes.

Gleiche Teile sind in allen Figuren mit denselben Bezugszeichen versehen.

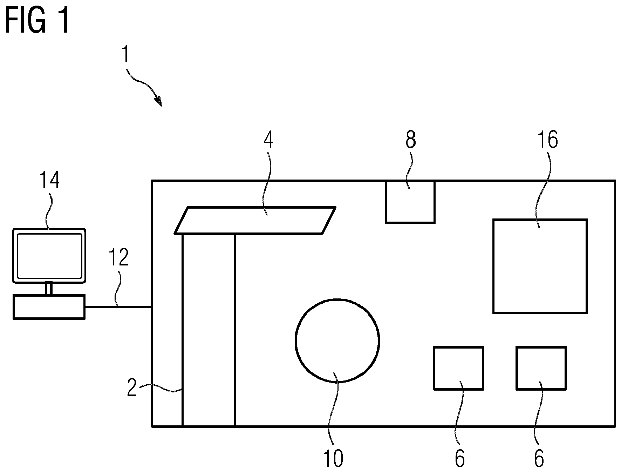

FIG 1 zeigt eine schematische Darstellung eines computergestützten Medizinprodukts mit einigen darin enthaltenen Bauteilen. Bei dem computergestützten Medizinprodukt handelt es sich um ein automatischen Analysegeräts 1. Hierbei werden nur die wichtigsten Bauteile stark vereinfacht dargestellt, um die grundsätzliche Funktion des automatischen Analysegeräts 1 zu erläutern, ohne hierbei detailliert die einzelnen Teile jedes Bauteils darzustellen.

Das automatische Analysegerät 1 ist dafür ausgebildet, vollautomatisch verschiedenste Analysen von Blut oder anderen Körperflüssigkeiten durchzuführen, ohne dass hierfür Aktivitäten eines Benutzers notwendig wären. Diese beschränken sich vielmehr auf Wartung oder Reparatur und Nachfüllarbeiten, wenn z.B. Küvetten oder Reagenzien nachgefüllt werden müssen.

Die Probengefäße werden dem automatischen Analysegerät 1 auf nicht näher dargestellten Schlitten in einer Zuführungsschiene 2 zugeführt. Informationen hinsichtlich der pro Probe durchzuführenden Analysen können hierbei beispielsweise mittels auf den Probengefäßen angebrachten Strichcodes übergeben werden, die in dem automatischen Analysegerät 1 ausgelesen werden. Aus den Probengefäßen werden in einer Pipettiereinrichtung 4 Aliquots mittels einer nicht näher dargestellten Pipettiernadel entnommen.

Die Aliquots werden ebenfalls nicht näher dargestellten Küvetten zugeführt, in denen die eigentlichen Analysen mittels verschiedenster Messgeräte 6, wie z.B. Photometern etc. durchgeführt werden. Die Küvetten werden aus einem Küvettenvorrat 8 entnommen. Zusätzlich können aus einem Reagenzbehältervorrat 10 mittels einer weiteren nicht weiter dargestellten Pipettiernadel weitere Reagenzien der jeweiligen Küvette zugeführt werden, die je nach durchzuführender Analyse erforderlich sind.

Der Transport der Küvetten innerhalb des automatischen Analysegeräts 1 erfolgt mit hier nicht näher dargestellten Transporteinrichtungen, wie z.B. Transferarmen, die in verschiedenste Raumrichtungen bewegbar sind und eine Greifeinrichtung zum Erfassen der Küvetten aufweisen. Der gesamte Prozess wird von einer zentralen Steuerungseinrichtung, wie z.B. einem über eine Datenleitung 12 angeschlossenen Rechner 14, gesteuert, unterstützt durch eine Vielzahl weiterer, nicht näher dargestellter elektronischer Schaltungen und Mikroprozessoren innerhalb des automatischen Analysegeräts 1 und seiner Bauteile. Der Rechner 14 weist hierbei vorteilhafterweise auch einen nichtflüchtigen Speicher in Form einer Festplatte, eines USB-Sticks oder ähnliches auf.

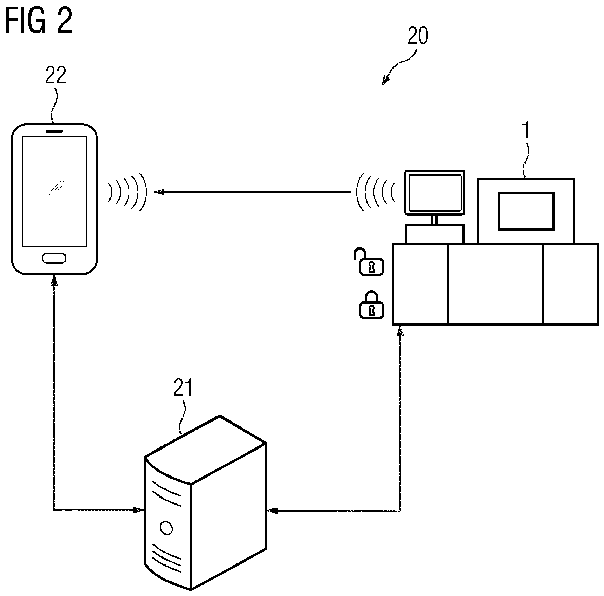

FIG 2 zeigt eine schematische Darstellung eines Systems 20 umfassend ein computergestütztes Medizinprodukt, das ein automatischer Analyzer 1 ist und einen Rechner 14 umfasst, eine Authentifizierungsdienst, der als Authentifizierungs-Server 21 ausgebildet ist und ein mobiles Endgerät 22, das ein Smartphone, ein Tablett-Computer oder eine Smartwatch ist. Das mobile Endgerät 22 ist so ausgebildet und konfiguriert, dass es von einem Benutzer, z.B. einem geschultem Laborant, bedient werden kann. Das System 20 umfasst folgende Komponenten. Eine speziell entwickelte mobile Anwendung ist auf dem mobilen Gerät des Benutzers auszuführen. Der Benutzer hat sich vorab mit einem Benutzernamen und einem Passwort bei einem dafür vorgesehenen Authentifizierungsserver anzumelden. Der Benutzer hat weiter bereits vorab über die mobile Anwendung eine Kopplung mit dem Zielsystem, also dem Geräterechner, vorgenommen. Der Geräterechner verfügt über eine Senderhardware für eine oder mehrerer drahtloser Technologien, z.B. NFC (Near Field Communication), BLE (Bluetooth Low Energy) oder Wi-Fi. Auf dem Geräterechner wird eine dedizierte Treibersoftware im Hintergrund ausgeführt, die mithilfe der verfügbaren drahtlosen Technologie das Gerät für die mobile Anwendung sichtbar macht. Hierbei wird ein spezielles Geräteidentifikationstoken bereitgestellt, welches die mobile Anwendung zum Entsperren des Benutzers benötigt. Die Signalstärke wird ausgewertet, um festzustellen, ob sich ein Benutzer in der Nähe befindet. Dadurch wird sichergestellt, dass der Geräterechner nur entsperrt wird, wenn sich der Benutzer in einem vordefinierten Abstand befindet. Die Sperrung erfolgt ebenfalls nach einem entsprechenden Muster. Ein Authentifizierungsserver ist für die Verwaltung der Benutzer und Geräte verantwortlich. Der Benutzer sowie die dedizierte Treibersoftware melden sich jeweils bei dieser Instanz an. Weiter erfolgt die Entsperrung eines Geräterechners über diesen Server. In Figur 2 sind schematisch über Pfeile entsprechende Datenverbindungen zwischen den einzelnen Komponenten des Systems dargestellt. Die Datenverbindung zwischen dem mobilen Endgerät und dem automatischen Analyzer 1 erfolgt dabei drahtlos.

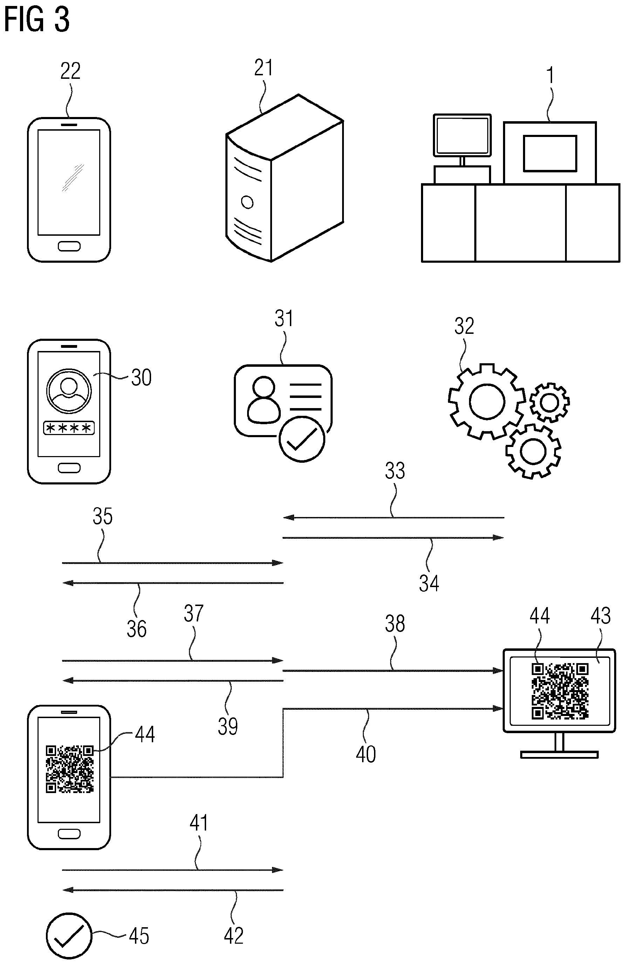

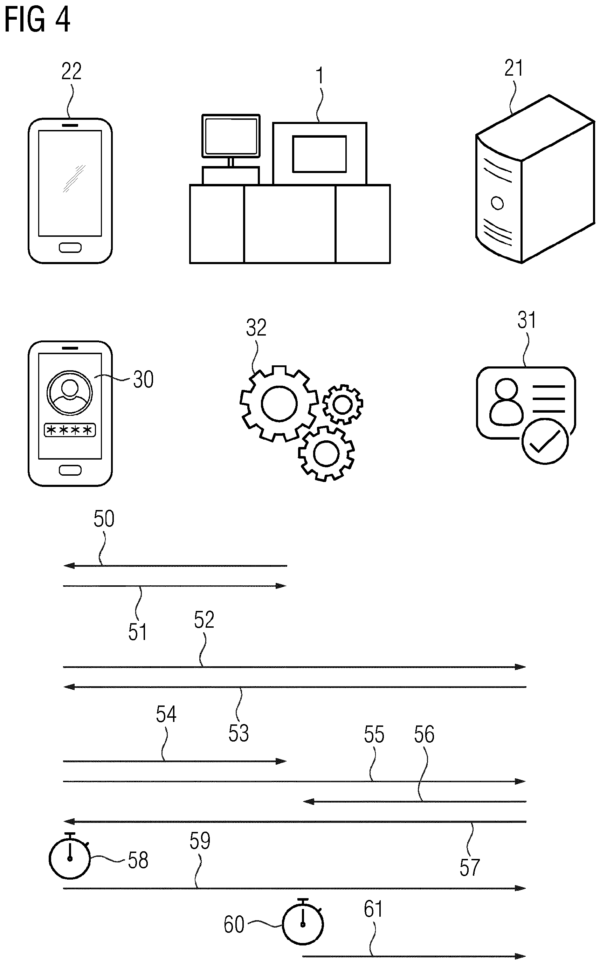

FIG 3 zeigt ein Ablaufdiagramm eines Verfahrens zur Gerätekopplung. Um sicherzustellen, dass der Benutzer autorisierten Zugriff auf das medizinische Gerät, wie z.B. den automatischen Analyzer 1, ein Diagnosesystem für bildgebende Verfahren oder ein sonstiges In-vitro Diagnosegerät hat, hat sich der Benutzer zunächst mittels unten abgebildeter Pairing-Methoden mit dem System zu verbinden. Dieser Vorgang muss einmalig pro Geräterechner und Benutzer durchgeführt werden. Im nächsten Schritt wird die dedizierte Treibersoftware mit Hilfe der Senderhardware das Gerät bekannt machen. Dabei wird das Geräteidentifikation-Token bekannt gegeben. Der Benutzer kann die mobile Anwendung verwenden, um die Systeme in der Umgebung zu erkennen. Wenn es sich um ein bereits gekoppeltes System handelt, kann sich der Benutzer anhand des in Figur 4 gezeigten Verfahrens am System anmelden, um den Geräterechner zu entsperren.

Das in FIG 3 dargestellt Ablaufdiagramm zeigt die drei Komponenten mobiles Endgerät 22, Authentifizierungs-Server 21 und ein automatisches Analysegerät 1 umfassend einen Rechner 14, auch als Geräterechner bezeichnet. Auf dem mobilen Endgerät 22 läuft eine mobile Anwendung 30. Der Authentifizierungs-Server 21 stellt eine entsprechende Web-Applikation 31 zur Verfügung. Auf dem Geräterechner (Rechner 14) läuft eine dedizierte Treibersoftware 32. Es erfolgt eine Geräteanmeldung seitens des Geräterechners mit der Web-Applikation 31 des Authentifizierungs-Servers 21 und der Authentifizierungs-Servers 21 stellt dem Geräterechner ein Geräteidentifikationstoken 34 zur Verfügung. Das mobile Endgerät 22 meldet sich mittels der mobilen Anwendung 30 bei der Web-Applikation 31 ebenfalls an (Anmeldung 35) und die Web-Applikation 31 stellt dem mobilen Endgerät 22 ein Sitzungstoken 36 zur Verfügung. Die mobile Anwendung 30 stellt eine Kopplungsanfrage 37 an die Web-Applikation 31, die daraufhin veranlasst, dass ein QR-Code 44 auf einer elektronischen Anzeigevorrichtung (Monitor 43) des Geräterechners angezeigt wird (Zeige QR-Code 38). Die Web-Applikation übermittelt eine Anfrage an die mobile Anwendung 30, den QR-Code mittels des mobilen Endgeräts 22 zu scannen (QR-Code scannen 40). Daraufhin scannt ein Benutzer mittels des mobilen Endgeräts 22 den auf dem Monitor 43 dargestellten QR-Code 44. Die mobile Anwendung 30 übermittelt den gescannten QR-Code 44 (gescannter QR-Code 41) an die Web-Applikation 31, die ihrerseits einen Geräteidentifikationstoken 42 an die mobile Anwendung 30 zur Verfügung stellt. Damit ist die Gerätekopplung abgeschlossen 45.

FIG 4 zeigt ein Ablaufdiagramm eines Verfahrens zum Entsperren eines Geräterechners eines computergestützten Medizinproduktes. Nachdem der Geräterechner entsperrt wurde, wird der Status der Verbindung von der dedizierten Treibersoftware sowie der mobilen Anwendung in einem vordefinierten Intervall von wenigen Minuten (z.B. 3 Minuten) an den Anmeldeserver gesendet. Hierbei wird unter anderem auch die Stärke des Signals von beide Seiten ermittelt und mitgeteilt. Anhand dieser Informationen kann der Login-Server feststellen, ob sich der Benutzer noch in unmittelbarer Nähe des Systems oder weiter entfernt befindet. Der Abstand kann je nach Gerätetypen variieren, beispielsweise von weniger Metern für ein kleineres Point-of-Care Gerät oder 10 Metern oder mehr für ein modulares Labor-Analysesystem oder eine Lobor-Automatisierungsstrasse. Mit Hilfe dieser Daten kann entschieden werden, ob der Geräterechner zu sperren ist oder entsperrt bleibt.

Das in FIG 4 dargestellt Ablaufdiagramm zeigt die drei Komponenten mobiles Endgerät 22, Authentifizierungs-Server 21 und ein automatisches Analysegerät 1 umfassend einen Rechner 14, auch als Geräterechner bezeichnet. Auf dem mobilen Endgerät 22 läuft eine mobile Anwendung 30. Der Authentifizierungs-Server 21 stellt eine entsprechende Web-Applikation 31 zur Verfügung. Auf dem Geräterechner (Rechner 14) läuft eine dedizierte Treibersoftware 32. Der Geräterechner stellt mittels der Treibersoftware eine Anfrage zur Werbung von Geräten (Gerätewerbung 50) über eine drahtlose Datenverbindung an die mobile Anwendung 30 des mobilen Endgeräts 22. Die mobile Anwendung 30 sucht nach Geräten (Nach Geräten suchen 51). Die mobile Anwendung 30 stellt eine Anfrage an die Web-Applikation 31, ob das Gerät gekoppelt wurde (Wurde Gerät gekoppelt? 52). Da das Gerät bereits gekoppelt ist, übermittelt die Web-Applikation eine entsprechende Mitteilung an die mobile Anwendung 30 (Gerät ist gekoppelt 53). Die mobile Anwendung 30 verbindet sich daraufhin mit der Senderhardware des Geräterechners über eine drahtlose Datenverbindung (Mit Senderhardware verbinden 54). Die mobile Anwendung 30 übermittelt eine Anfrage, das Gerät zu entsperren an die Web-Applikation 31 (Gerät entsperren 55). Mittels der Web-Applikation 31 erfolgt die Anmeldung als Benutzer an dem Geräterechner und die Entsperrung des Geräterechners bzw. des automatischen Analysegeräts 1 (Mit Benutzer anmelden und entsperren 56). Weiter übermittelt die Web-Applikation 31 an die mobile Anwendung, dass der Geräterechner bzw. das automatische Analysegerät 1 entsperrt wurde (Geräterechner entsperrt 57). In vordefinierten Zeitintervallen 58, 60 sendet die mobile Anwendung 30 und der Geräterechner jeweils den aktuellen jeweiligen Verbindungsstatus an die Web-Applikation 31 (Sende Verbindungsstatus 59, 61).

**BEZUGSZEICHENLISTE**

1      automatisches Analysegerät
2      Zuführungsschiene
4      Pipettiereinrichtung
6      Messgerät
8      Küvettenvorrat
10     Reagenzbehältervorrat
12     Datenleitung
14     Rechner
20     System
21     Authentifizierungs-Server
22     mobiles Endgerät
30     mobile Anwendung
31     Web-Applikation
32     Treibersoftware
33     Geräteanmeldung
34     Geräteidentifikationstoken
35     Anmeldung
36     Sitzungstoken
37     Kopplungsanfrage
38     Zeige QR-Code
40     QR-Code scannen
41     gescannter QR-Code
42     Geräteidentifikationstoken
43     Monitor
44     QR-Code
45     Gerätekopplung abgeschlossen
50     Gerätewerbung
51     Nach Geräten suchen
52     Wurde Gerät gekoppelt?
53     Gerät ist gekoppelt
54     Mit Sendehardware verbinden
55     Gerät entsperren
56     Mit Benutzer anmelden und entsperren
57     Geräterechner entsperrt 60
58     Zeitintervall
59     Sende Verbindungsstatus
60     Zeitintervall
61     Sende Verbindungsstatus

**Patentansprüche**

1.  Verfahren zum automatischen Entsperren und/oder Sperren eines computergestützten Medizinprodukts in einem System, das System umfassend das computergestützte Medizinprodukt und ein mobiles Endgerät, wobei das mobile Endgerät eine erste drahtlose Kommunikationsvorrichtung zum drahtlosen Senden und Empfangen von Daten umfasst und wobei mittels des mobilen Endgeräts eine mobile Anwendungssoftware ausführbar ist, wobei das Medizinprodukt einen Gerätecomputer umfasst und wobei der Gerätecomputer eine zweite drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung des Medizinprodukts eine drahtlose Kommunikationsverbindung zu der ersten Kommunikationsvorrichtung des mobilen Endgeräts aufbauen kann, **dadurch gekennzeichnet, dass** das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der zweiten Kommunikationsvorrichtung das Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der Kommunikationsvorrichtung das Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

2. Verfahren nach Anspruch 1, wobei das System weiter einen Authentifizierungsdienst umfasst, wobei eine drahtlose Kommunikationsverbindung das mobilen Endgeräts mit der ersten Kommunikationsvorrichtung zu dem Authentifizierungsdienst aufbaubar ist und wobei eine Kommunikationsverbindung zwischen dem Gerätecomputer des Medizinprodukts und dem Authentifizierungsdienst aufbaubar ist und wobei die Entsperrung und/oder Sperrung des Medizinprodukts mittels des Authentifizierungsdienst erfolgt, wobei der Authentifizierungsdienst bevorzugt einen Authentifizierungsserver umfasst oder ein Authentifizierungsserver ist.

3. Verfahren nach Anspruch 2, wobei eine Authentifizierung des mobilen Endgeräts und/oder des Gerätecomputers des Medizinprodukts bei dem Authentifizierungsdienst erfolgt.

4. Verfahren nach Anspruch 3, wobei die Authentifizierung des mobilen Endgeräts über eine drahtlose Kommunikationsverbindung das mobilen Endgeräts mit der ersten Kommunikationsvorrichtung zu dem Authentifizierungsdienst erfolgt.

5. Verfahren nach Anspruch 3, wobei die Authentifizierung des Gerätecomputers des Medizinprodukts über eine Kommunikationsverbindung des Gerätecomputers des Medizinprodukt zu dem Authentifizierungsdienst erfolgt.

6. Verfahren nach einem der vorhergehenden Ansprüche, wobei das mobile Endgerät ein Smartphone oder einen tragbaren Computer, bevorzugt einen Tablett Computer, umfasst oder das mobile Endgerät ein Smartphone oder ein tragbarer Computer, bevorzugt ein Tablett Computer, ist.

7. Verfahren nach einem der vorhergehenden Ansprüche, wobei das Medizinprodukt einen automatischen Analysator, bevorzugt einen in-vitro Analysator für medizinische Proben, bevorzugt für Blut umfasst.

8. Verfahren nach einem der vorhergehenden Ansprüche, wobei der erste Grenzwert einem ersten Abstand zwischen dem mobile Endgerät und dem Medizinproduktzugeordnet zugeordnet ist oder entspricht, wobei der erste Abstand bevorzugt weniger als 10 Meter, besonders bevorzugt weniger als 5 Meter beträgt.

9. Verfahren nach einem der vorhergehenden Ansprüche, wobei der zweite Grenzwert einem zweiten Abstand zwischen dem mobile Endgerät und dem Medizinprodukt zugeordnet ist oder entspricht, wobei der zweite Abstand bevorzugt weniger als 10 Meter, besonders bevorzugt weniger als 5 Meter beträgt.

10. Verfahren nach einem der vorhergehenden Ansprüche, wobei das erste Zeitintervall bevorzugt kleiner als 3 Minuten, besonders bevorzugt kleiner als 1 Minute ist.

11. Verfahren nach einem der vorhergehenden Ansprüche, wobei der erste Grenzwert kleiner ist als der zweite Grenzwert.

12. Verfahren nach einem der vorhergehenden Ansprüche, wobei das Entsperren die Freigabe einer graphischen Bedienoberfläche einer elektronischen Anzeigevorrichtung des Medizinproduktes und/oder die Freigabe einer Benutzerschnittstelle des Medizinproduktes für die Eingabe von Befehlen umfasst.

13. Verfahren nach einem der vorhergehenden Ansprüche, wobei das Sperren die Blockierung einer graphischen Bedienoberfläche einer elektronischen Anzeigevorrichtung des Medizinproduktes und/oder die Blockierung einer Benutzerschnittstelle des Medizinproduktes für die Eingabe von Befehlen umfasst.

14. Medizinprodukt umfassend einen Gerätecomputer, wobei der Gerätecomputer eine erste drahtlose Kommunikationsvorrichtung zum drahtlosen Senden und Empfangen von Daten umfasst, wobei auf dem Gerätecomputer eine Treibersoftware ausführbar ist, die mittels der zweiten Kommunikationsvorrichtung das Medizinprodukts eine drahtlose Kommunikationsverbindung zu einer ersten drahtlose Kommunikationsvorrichtung eines mobilen Endgeräts aufbauen kann, wobei das mobile Endgerät die erste drahtlose Kommunikationsvorrichtung zum drahtlosen Senden und Empfangen von Daten umfasst und wobei mittels des mobilen Endgeräts eine mobile Anwendungssoftware ausführbar ist, **dadurch gekennzeichnet, dass** der Gerätecomputer so konfiguriert ist, dass das Medizinprodukt automatisch von einem gesperrtem Zustand in einen entsperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der zweiten Kommunikationsvorrichtung das Medizinprodukts empfangene Signalstärke des Signals der ersten Kommunikationsvorrichtung des mobilen Endgeräts einen vorbestimmten ersten Grenzwert überschreitet und/oder das Medizinprodukt automatisch von einem entsperrtem Zustand in einen gesperrten Zustand versetzt wird, wenn die erste Kommunikationsvorrichtung des mobilen Endgeräts eine drahtlose Verbindung mit der zweiten Kommunikationsvorrichtung das Medizinprodukts aufgebaut hat und die von der Kommunikationsvorrichtung das Medizinprodukts empfangene Signalstärke des Signals der ersten Kommunikationsvorrichtung des mobilen Endgeräts einen vorbestimmten zweiten Grenzwert unterschreitet und/oder wenn die drahtlose Verbindung länger als ein vorbestimmtes erstes Zeitintervall unterbrochen ist.

15. Medizinprodukt nach Anspruch 14, wobei das Medizinprodukt einen automatischen Analysator, bevorzugt einen in-vitro Analysator für medizinische Proben, bevorzugt für Blut umfasst.

16. Verwendung eines Verfahrens nach einem der Ansprüche 1 bis 13 in einem System, das System umfassend ein computergestützte Medizinprodukt und ein mobiles Endgerät.

FIG 1

FIG 2

# FIG 3

# FIG 4

Europäisches
Patentamt

European
Patent Office

Office européen
des brevets

## EUROPÄISCHER RECHERCHENBERICHT

Nummer der Anmeldung

EP 20 15 4624

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2017/325091 A1 (FREEMAN GARY A [US] ET AL) 9. November 2017 (2017-11-09)<br>* Absätze [0002], [0006] - [0010], [0016], [0017], [0042] - [0048], [0067], [0120] - [0125], [0135] - [0142], [0183] *<br>* Absätze [0219], [0237] *<br>----- | 1-16 | INV.<br>G06F21/35<br>H04L9/32<br>H04L29/06<br>H04W12/08<br>G01N35/00<br>G06F21/62<br>A61B5/15 |
| Y | US 2017/302659 A1 (SHTEINGART HANAN [IL] ET AL) 19. Oktober 2017 (2017-10-19)<br>* Absätze [0015], [0038] - [0045], [0052] - [0059] *<br>----- | 1-16 | |
| A | EP 3 087 771 A1 (ABBOTT DIABETES CARE INC [US]) 2. November 2016 (2016-11-02)<br>* Absätze [0007], [0048] - [0052], [0058], [0065] - [0067], [0080] - [0082], [0119] - [0123] *<br>----- | 1-16 | |

| RECHERCHIERTE SACHGEBIETE (IPC) |
|---|
| G06F<br>H04L<br>H04W<br>G01N<br>A61B |

Der vorliegende Recherchenbericht wurde für alle Patentansprüche erstellt

| Recherchenort | Abschlußdatum der Recherche | Prüfer |
|---|---|---|
| München | 8. Juli 2020 | Herzog, Till-Henning |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................
& : Mitglied der gleichen Patentfamilie, übereinstimmendes Dokument

EPO FORM 1503 03.82 (P04C03)

EP 3 859 573 A1

**ANHANG ZUM EUROPÄISCHEN RECHERCHENBERICHT
ÜBER DIE EUROPÄISCHE PATENTANMELDUNG NR.**

EP 20 15 4624

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-07-2020

| Im Recherchenbericht angeführtes Patentdokument | Datum der Veröffentlichung | Mitglied(er) der Patentfamilie | Datum der Veröffentlichung |
|---|---|---|---|
| US 2017325091 A1 | 09-11-2017 | US 2017325091 A1<br>US 2020084629 A1 | 09-11-2017<br>12-03-2020 |
| US 2017302659 A1 | 19-10-2017 | CN 109076076 A<br>EP 3446457 A1<br>US 2017302659 A1<br>WO 2017184388 A1 | 21-12-2018<br>27-02-2019<br>19-10-2017<br>26-10-2017 |
| EP 3087771 A1 | 02-11-2016 | EP 3087771 A1<br>US 2015207796 A1<br>US 2017093869 A1<br>US 2019190913 A1<br>WO 2015100109 A1 | 02-11-2016<br>23-07-2015<br>30-03-2017<br>20-06-2019<br>02-07-2015 |

Für nähere Einzelheiten zu diesem Anhang : siehe Amtsblatt des Europäischen Patentamts, Nr.12/82